# EUROPEAN PATENT APPLICATION

(11) **EP 4 130 737 A1**
(43) Date of publication of application: **08.02.2023**
(21) Application number: 21781639.6
(22) Date of filing: 30.03.2021
(51) Int. Cl.: G01N 33/02

(54) **OUTPUT DEVICE, INFORMATION PROCESSING SYSTEM, AND PROGRAM**

(30) Priority: 31.03.2020 JP 2020065078
(71) Applicant: Daikin Industries, Ltd., Osaka-shi, Osaka 530-0001 (JP)
(72) Inventor: SATOU, Kiichirou, Osaka-shi, Osaka 530-8323 (JP); MATSUI, Hidenori, Osaka-shi, Osaka 530-8323 (JP); TANNO, Shouichi, Osaka-shi, Osaka 530-8323 (JP); TANAKA, Naohiro, Osaka-shi, Osaka 530-8323 (JP)
(74) Representative: Hoffmann Eitle
(86) International application number: PCT/JP2021/013471
(87) International publication number: WO 2021/200904

(57) **Abstract**

Ripening degree and contamination degree of perishables are specified by using a sensor, and states and handling of perishables are determined based on the specified ripening degree and contamination degree. A perishable management device 300 includes: a ripening degree specifying section 320 configured to specify ripening degree of perishables; a contamination degree specifying section 330 configured to specify contamination degree of perishables; a determination section 340 and a proposed information specifying section 360 configured to specify information related to perishables acquired by ripening degree and contamination degree of perishables; and a determination result output section 380 configured to output the specified information.

## Description

### Technical Field

The present disclosure relates to an output device, an information processing system and a program.

### Background Art

Patent Literature 1 discloses a sealable container and in an inspection system provided with detection device that detects a substance emitted by specimens in accordance with an inspection operation, when the specimens and the detection device are stored in the container and the inspection operation is performed, the detection device stored in the container detects the substance emitted by the specimens stored in the container. It also discloses that a quality of the specimens is judged on the basis of the information related to the substance emitted by the specimens detected by the detection device.

### Citation List

### Patent Literature

Patent Literature 1: International Publication WO2017-130775

### Summary of Invention

### Technical Problem

If determining states and handling of perishable food only by ripening degree, it is impossible to provide appropriate information when the perishable food is decayed due to molds and bacteria causing food poisoning are attached to the perishable food. On the other hand, if determining states and handling of perishable food only by contamination degree, it is impossible to provide appropriate information when the perishable food is not ripened.

It is an object of the present disclosure to determine precisely states and handling of perishables.

### Solution to Problem

An output device of the present disclosure is an output device for information on perishables including: a ripening degree specifying section configured to specify ripening degree of perishables; a contamination degree specifying section configured to specify contamination degree of the perishables; and an output section configured to output information related to the perishables acquired by the ripening degree and the contamination degree of the perishables.

This output device can determine precisely states and handling of the perishables.

Here, the output device may be configured to output at least one of information related to peak ripeness of the perishables, information related to preservation of the perishables and information related to ways of eating the perishables.

This makes it possible to output information complementary related to "peak ripeness", "preservation methods" and "ways of eating" as information related to perishables.

Also, the output device may be configured to output information related to peak ripeness of the perishables, and/or information related to preservation of the perishables according to acquired information related to ways of eating the perishables.

This makes it possible to output information on "peak ripeness" and "preservation methods" complementary related to particular "ways of eating" regarding perishables.

Moreover, the output device may be configured to output information related to ways of eating the perishables and/or information related to preservation of the perishables according to acquired information related to time to eat the perishables.

This makes it possible to output information on "ways of eating" and "preservation methods" complementary related to particular "time to eat" regarding perishables.

Furthermore, the output device may be configured to output information related to peak ripeness of the perishables, and/or information related to ways of eating the perishables according to acquired information related to preservation methods of the perishables.

This makes it possible to output information on "peak ripeness" and "ways of eating" complementary related to particular "preservation methods" regarding perishables.

In addition, this output device may include: an acquisition section configured to acquire types of the perishables; wherein the output section may be configured to output information related to the perishables according to types of the perishables acquired by the acquisition section.

This makes it possible to determine precisely states and handling of the perishables based on ripening degree and contamination degree which are acquired according to types of perishables subjected to process, also for plural types of perishables.

Further, in this output device, the ripening degree specifying section may be configured to detect components related to ripening emitted by the perishables by a sensor using biological components.

This makes it possible to detect components related to ripening emitted by the perishables with higher responsiveness compared to detection by electronic devices.

Also, in this output device, the contamination degree specifying section may be configured to detect components related to contamination emitted by the perishables by a sensor using biological components.

This makes it possible to detect components related to contamination emitted by the perishables with higher responsiveness compared to detection by electronic devices.

Moreover, an information processing system of the present disclosure is an information processing system for perishables including: a ripening degree acquisition section configured to acquire ripening degree of perishables; a contamination degree acquisition section configured to acquire contamination degree of the perishables; and an output section configured to output proposed information on the perishables for users based on the ripening degree and the contamination degree of the perishables.

This information processing system makes it possible to output more appropriately proposed information on the perishables for users.

Here, the output section may be configured to output the proposed information including different contents according to users.

This makes it possible to provide more appropriate information for each user even if a process is related to same perishables.

Furthermore, a program of the present disclosure is a program causing a computer to implement: a function configured to acquire ripening degree of the perishables; a function configured to acquire contamination degree of the perishables; and a function configured to output information related to the perishables acquired by the ripening degree and the contamination degree of the perishables.

A computer with this program installed makes it possible to determine precisely states and handling of the perishables.

### Brief Description of Drawings

FIG. 1 is an entire configuration of a perishable management system according to an embodiment.
FIG. 2 is a configuration of a detection device.
FIG. 3 is an example of a hardware configuration of a perishable management device.
FIG. 4 is an example of a functional configuration of the perishable management device.
FIG. 5 is relationship between changes in freshness and ripening degree.
FIG. 6 is an example of a configuration of perishable database.
FIG. 7 is an example of a configuration of a UI (user interface) screen for receiving inputs of designated conditions.
FIG. 8 is an example of a configuration of a UI screen for presenting proposed information.

### Description of Embodiments

Embodiments of the present invention will be described below in detail with reference to the attached drawings.

### <System Configuration>

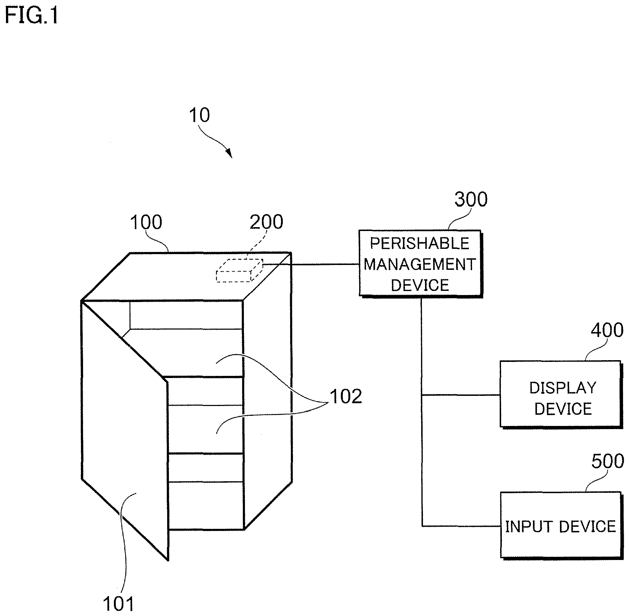

FIG. 1 is an entire configuration of a perishable management system according to an embodiment. A perishable management system 10 of the embodiment includes a storage cabinet 100, a detection device 200, a perishable management device 300, a display device 400, and an input device 500.

The storage cabinet 100 is a device containing and storing perishables. The storage cabinet 100 shown in FIG. 1 is a box-shaped device having a space for containing perishables inside, and perishables can be taken in and out of the internal space by opening a door 101. Also, the storage cabinet 100 may be configured so that when the door 101 is closed, the space inside the storage cabinet 100 is sealed and the storage cabinet 100 is separated from external environment. The space inside the storage cabinet 100 is divided by a storage shelf 102 so that perishables can be sorted and stored. The storage cabinet 100 may be provided with an environmental adjustment device (not shown) which adjusts internal environment by controlling temperature, humidity, air pressure, components of air that fulfill the space inside and the like. Specifically, the storage cabinet 100 can be assumed to be something that contains perishables in refrigerated environment, in frozen environment, at normal temperature or the like. Different environment may be set for each area divided by the storage shelf 102 when environment inside the storage cabinet 100 is controlled in the way described above.

The detection device 200 is provided inside the storage cabinet 100, and acquires data indicating states of the space inside the storage cabinet 100. In the present embodiment, ripening degree and contamination degree of perishables contained in the storage cabinet 100 are determined by using data acquired by the detection device 200. For that reason, the detection device 200 capable of acquiring data used for determining ripening degree and contamination degree of perishables is employed. For example, the detection device 200 may be a detection section detecting air quality by using biological components. There are also cases where different types of data are used for determining ripening degree and contamination degree according to types of perishables and causes of contamination of perishables contained in the storage cabinet 100. Relationship between data that should be acquired and the detection device 200 is described later.

The perishable management device 300 manages information on perishables contained in the storage cabinet 100. Particularly, the perishables management device 300 determines both ripening degree and contamination degree of perishables contained in the storage cabinet 100 by using data acquired by the detection device 200. Then, the perishables management device 300 specifies information related to perishables, based on both acquired ripening degree and contamination degree, more precisely than the case using either ripening degree or contamination degree. Furthermore, the perishable management device 300 outputs information corresponding to specific conditions regarding perishables. The perishable management device 300 is implemented by, for example, a personal computer, a smartphone, or an embedded computer provided in the storage cabinet 100. The perishables management device 300 may be configured to be provided together with the storage cabinet 100, or provided separately from the storage cabinet 100 acquiring data from the detection device 200 via a communication line. The communication line may be wired or wireless.

The display device 400 is a device displaying a screen such as an operation screen and an information presenting screen. The display device 400 displays an information presenting screen presenting information related to perishables acquired from the perishables management device 300 to users, an operation screen receiving an operation by users and the like. The display device 400 may be, for example, a liquid crystal display, etc. When the management device 300 described above is configured with a personal computer, the display device 400 may be a display device of this personal computer. Also, the display device 400 may be an individual display device provided to the storage cabinet 100 when the management device 300 is configured with an embedded computer.

The input device 500 is a device receiving input operations by users. The input device 500 receives operations done by users according to an operation screen displayed on the display device 400. For example, devices such as a keyboard and a mouse are used as the input device 500. Also, a touch panel as a user interface may be configured with a touch sensor as the input device 500 in combination with a liquid crystal display which is the display device 400.

### <Configuration of Detection Device 200>

The detection device 200 acquires data to specify ripening degree and contamination degree of perishables contained in the storage cabinet 100 based on air quality inside the storage cabinet 100. Here, an index for air quality is percentages of particular components in air and so on. Particular components can be assumed to be specific air components such as ethylene gas and carbon dioxide, and odorant components. The detection device 200 detects ethylene gas or the like which perishables emit according to ripening degree of perishables contained in the storage cabinet 100. The detection device 200 also detects smells emitted by molds on perishables contained in the storage cabinet 100. Then, ripening degree and contamination degree of perishables are specified based on these detection results.

A configuration of the detection device 200 and a mechanism for acquiring data may employ various things. Here, a device which detects air quality by using biological components is described as an example of a specific configuration of the detection device 200. Air inside the storage cabinet 100 is collected by a mechanism collecting air (not shown) and sent to the detection device 200. The detection device 200 itself may be provided outside the storage cabinet 100.

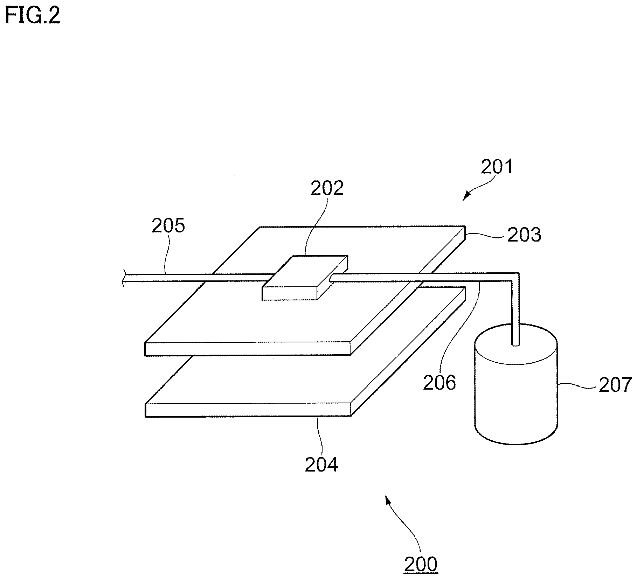

FIG. 2 is a configuration of the detection device 200. The detection device 200 detects air quality collected from the storage cabinet 100 by using biological components. The detection device 200 is provided with a cell support section 201 supporting cells for protein expression of olfactory receptor of insects (hereinafter, referred to as receptor expression cells).

The cell support section 201 is provided with a cell container 202 that contains receptor expression cells, and a substrate 203 supporting the cell container 202. The substrate 203 is composed of transparent materials such as glass. Also, the detection device 200 is provided with a sensor 204 that detects lights emitted by receptor expression cells and outputs signals. The signals from the sensor 204 are output to the perishable management device 300.

The receptor expression cells can be created by general genetic engineering methods. Specifically, genes that code olfactory receptor protein of the insects against specific odorants and genes that code fluorescent protein to confirm that the odorants are combined with the olfactory receptor protein are embedded with a vector for an expression of insects' cultivation cells. Then, receptor expression cells are created by transfecting the vector for the expression of the insect's cultivation cells to host cells.

Insects are, for example, drosophila melanogaster, anopheles and silkworm moth. More than 100 kinds of olfactory receptor proteins are specified from those insects. Olfactory receptor proteins have higher responsiveness towards odorants, for example, phenethyl alcohol, methyl benzoate, ethyl benzoate, benzyl alcohol, methyl salicylate, benzaldehyde, pentanal, hexanal, E2-hexenal, 2-heptanone, 6-methyl-5-hepten-2-one, and 2-methylphenol.

In receptor expression cells, when odorants subjected to detect combine with receptors, ions flow in receptor expression cells, and receptor expression cells combined with odorants emit light. The configuration of the detection device 200 is not limited and may be implemented by known configurations.

In the configuration shown in FIG. 2, air collected in the storage cabinet 100 is provided to the cell container 202 from an upstream-side flow path 205 disposed on the cell support section 201. After that, the air passes through a downstream-side flow path 206 and is released from an emission container 207 to the outside. It should be noted that although, in the configuration above, air from the storage cabinet 100 is directly provided to the cell container 202, it is not limited to this, and air may be dissolved in a liquid solution and the liquid solution including the dissolved air may be provided to the cell container 202.

The sensor 204 is provided back side of the cell support section 201 for example. Specifically, the sensor 204 sandwiches the substrate 203 and is provided on the opposite side of the cell container 202. The sensor 204 is composed of, for example, a CCD image sensor and a CMOS image sensor. Photoelectric conversion elements are placed on the sensor 204 in a two-dimensional array state. The sensor 204 acquires an image of the cell container 202 by using photoelectric conversion elements. The image acquired by the sensor 204 is sent to the perishable management device 300. In this example, the perishable management device 300 determines, by analyzing this image, whether air collected from the storage cabinet 100 includes predetermined specified substances related to ripening degree and contamination degree of the contained perishables.

It should be noted that although the example of the configuration shown in FIG. 2 includes one cell container 202, different kinds of receptor expression cells may be contained in each cell container 202 along with preparing plural cell containers 202. In this case, it is possible to detect plural types of substances. Further, the example above descried a detection device 200 using biological components, but the detection device 200 may be composed of an electronic device. Note that a detection device 200 using biological components can detect components related to ripening and contamination emitted by perishables with higher responsiveness compared to using electronic devices.

### <Configuration of Perishable Management Device 300>

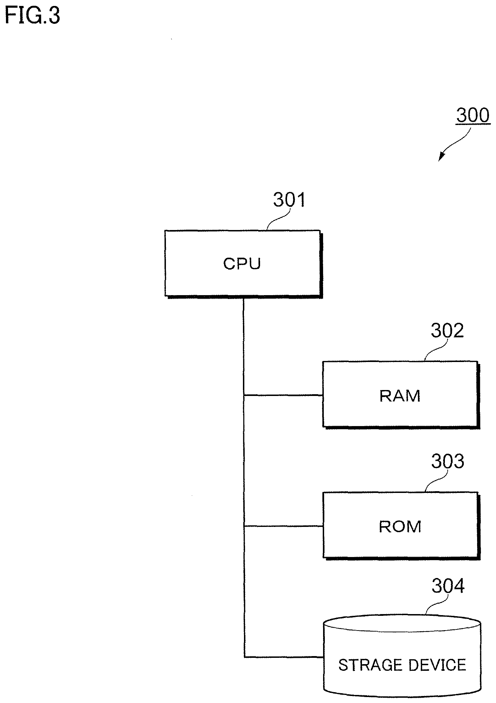

FIG. 3 is an example of a hardware configuration of the perishable management device 300. A computer that implements the perishable management device 300 is composed of a central processing unit (CPU) 301 as a calculation section, a random access memory (RAM) 302 as a storage section, a read only memory (ROM) 303, and a storage device 304. The RAM 302 is a main memory and used as a working memory for the CPU 301 to perform calculation processing. The ROM 303 keeps programs and data such as previously prepared set values, and the CPU 301 directly reads programs and data from the ROM 303 and can perform processing. The storage device 304 is a storing section for programs and data. Programs are stored in the storage device 304, and the CPU 301 reads the programs stored in the storage device 304 into the main memory and runs them. Also, processing results by the CPU 301 are stored in the storage device 304. Magnetic disc devices and solid state drives (SSD), for example, are used as a storage device 304. Note that the description of a hardware configuration here is assumed to use one single computer, but each function of the perishables management device 300 described later may be implemented dispersedly by plural computers.

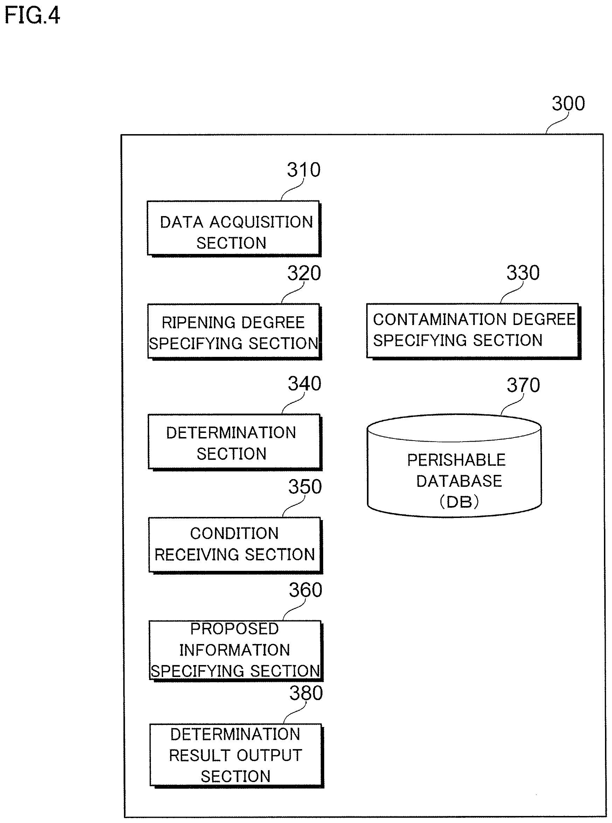

FIG. 4 is an example of a functional configuration of the perishables management device 300. The perishables management device 300 includes: a data acquisition section 310, a ripening degree specifying section 320, a contamination degree specifying section 330, a determination section 340, a condition receiving section 350, a proposed information specifying section 360, a perishable database (DB) 370, and a determination result output section 380. The data acquisition section 310, the ripening degree specifying section 320, the contamination degree specifying section 330, the determination section 340, the condition receiving section 350, the proposed information specifying section 360, and the determination result output section 380 among the functions are performed, for example in the computer shown in FIG. 3, by the CPU 301 executing programs. Also, the perishable database 370 is implemented, for example, by the storage device 304 of the computer shown in FIG. 3.

The data acquisition section 310 is a function that acquires data relating to air inside the storage cabinet 100 acquired by the detection device 200. The data acquisition section 310 receives data sent from the detection device 200 via interface connected to the detection device 200.

The ripening degree specifying section 320 is a function that determines ripening degree of perishables contained in the storage cabinet 100 based on the data acquired by the data acquisition section 310. The ripening degree specifying section 320 of the perishables management device 300 is an example of a ripening degree specifying section. Ripening degree is one of indexes for perishables quality and is what is called an index for peak ripeness. Although there are various indexes for perishables quality, here, ripening degree is described in comparison with freshness.

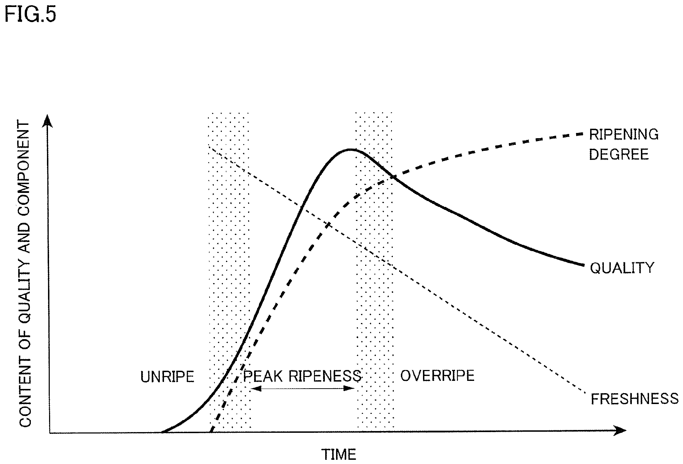

FIG. 5 is relationship between changes in freshness and ripening degree. As shown in FIG. 5, freshness is 100% at harvest and decreases as time goes by (refer to the dashed line graph in FIG. 5). On the other hand, the way ripening degree changes over time differs depending on types of perishables. For example, ripening degree of climacteric fruits (avocado, banana, tomato, etc) increases by ripening after harvest (refer to the dashed-and-dotted line graph in FIG. 5). Thus, quality of this type of perishables improves together with rise of ripening degree after harvest and is lowered along with deterioration of freshness when the increase in ripening degree hits a peak after a certain time (refer to the solid-line graph in FIG. 5). Therefore, so-called peak ripeness for this type of perishables is from time when ripening after harvest has progressed to some extent to time when freshness is lowered and right before those perishables become overripe. It should be noted that changes in quality over time as described above do not happen to all perishables. For example, since non-climacteric fruits such as pear do not ripen after harvest, quality is the highest right after harvest and just decreases as time passes. Also note that preservation method is affected by progress rates of ripening degree of perishables. For example, it is possible to slow down ripening progress by low-temperature preservation (refrigeration, freezing and others) or controlling components of air inside the storage cabinet 100 depending on types of perishables.

Methods for determining ripening degree by ripening degree specifying section 320 may be different according to types of perishables subjected to determine. An example is that different biological components may be used according to types of perishables subjected to determine when ripening degree of perishables is determined by using biological components as the detection device 200 described with FIG. 2 does. Specifically, for example, insect cells of drosophila may be utilized to determine ripening degree of meat whereas insect cells of nicrophorus may be utilized to determine ripening degree of fruits.

Returning to FIG. 4, the contamination degree specifying section 330 is a function to determine contamination degree of perishables contained in the storage cabinet 100 based on data acquired by the data acquisition section 310. The contamination degree specifying section 330 of the perishable management device 300 is an example of a contamination degree specifying section. Contamination degree simply proceeds over time usually after causes of contamination such as bacteria occur in perishables. However, low-temperature preservation (refrigeration, freezing and others) makes it possible to prevent causes of contamination from spreading and slow down progress of contamination degree. Methods for determining contamination degree may differ according to causes of contamination to be determined. An example is that different biological components may be used according to causes of contamination to be determined when contamination degree of perishables is determined by using biological components such as the detection device 200 described with FIG. 2 does. Specifically, for example, insect cells of drosophila may be utilized to determine contamination degree by molds whereas insect cells of anopheles or bedbug may be utilized to determine contamination degree by bacteria causing food poisoning.

The determination section 340 is a function to determine information related to perishables based on information on ripening degree of perishables determined by the ripening degree specifying section 320 and contamination degree of perishables determined by the contamination degree specifying section 330. Specifically, for example, information related to peak ripeness of perishables, information related to preservation of perishables, information related to ways of eating perishables, and the like are determined as information related to perishables.

The determination section 340 estimates a period that peak ripeness of perishables is being kept based on information on transition of ripening degree over time in perishables subjected to determine and current ripening degree of perishables determined by the ripening degree specifying section 320. Here, the period that peak ripeness is being kept is a period until ripening degree proceeds and become overripe. In the case where progress of ripening degree is different according to preservation methods, the period that peak ripeness is being kept is estimated for each preservation method.

Moreover, the determination section 340 estimates a period that perishables become inedible based on information on transition of contamination degree over time in perishables subjected to determine and current contamination degree of perishables determined by the contamination degree specifying section 330. Here, depending on types of perishables, there may be states where they cannot be eaten raw but can be eaten if heated according to progress of contamination degree. Therefore, regarding such perishables, it is estimated, for each, that a time when they become inedible if raw and a time when they become inedible even if heated. Furthermore, in the case where progress of contamination degree is different according to preservation methods, a time when perishables become inedible is estimated for each preservation method.

As described above, the determination section 340 determines information related to perishables by using each of information on ripening degree of perishables and information on contamination degree of perishables. Since ripening degree and contamination degree are different indexes relating to perishables quality, determination based on only one of them may result in overlooking matters related to perishables quality. For example, in the case where contamination of perishables is also progressing because bacteria that cause contamination are attached to them although perishables themselves are ripening, precision in determining information related to perishables is decreased if determined based only on ripening degree. Furthermore, in the case where perishables are stored in an aseptic condition, precision in determining information related to perishables is decreased if determined based only on contamination degree. Therefore, it is possible to determine the information more precisely by determining based on both ripening degree and contamination degree than determining based only one of them.

The condition receiving section 350 is a function to receive conditions that users designate to acquire information related to perishables. The condition receiving section 350 is an example of an acquisition section. Conditions are input by users operating the input device 500 for example. Here, the condition receiving section 350 receives a designation of conditions (hereinafter, referred to as designated conditions) to acquire at least any one of information: information related to peak ripeness of perishables, information related to preservation of perishables and information related to ways of eating perishables.

As a specific example, the condition receiving section 350 receives inputs of at least one of conditions: "ways of eating", "when to eat", and "preservation methods" as designated conditions. For example, conditions such as raw food and heating are designated as "ways of eating". Designated conditions may be a combination of plural conditions. For example, users may designate a combination of "ways of eating" and "preservation methods".

The proposed information specifying section 360 is a function to specify proposed information according to designated conditions received by the condition receiving section 350. Information related to peak ripeness of perishables, information related to preservation methods of perishables and information related to ways of eating perishables, described above, are information complementary related each other. Therefore, by designating any of the information as designated conditions, proposed information becomes information which is not designated by designated conditions among information related to perishables. For example, a period that peak ripeness suitable for designated ways of eating is being kept is decided for each preservation method if "ways of eating" are designated as designated conditions for certain perishables. Also, a combination of ways of eating that are possible even during a designated time and preservation methods is decided if "when to eat" is designated as designated conditions. Moreover, how long peak ripeness for perishables stored by designated preservation methods is decided for each way of eating if "preservation methods" are designated as designated conditions. Furthermore, a period that peak ripeness is being kept by designated ways of eating and preservation methods is decided if "ways of eating" and "preservation methods" are designated as designated conditions. Ways of eating, which are possible when perishables stored in designated preservation methods are eaten during a designated time, are decided if "when to eat" and "preservation methods" are designated as designated conditions. Preservation methods are decided to eat perishables in designated ways of eating during a designated time if "ways of eating" and "when to eat" are designated as designated conditions. The proposed information specifying section 360 specifies, as described above, information related to perishables specified according to designated information as proposed information presenting to users.

Here, proposed information may be presented as different contents depending on users. For example, regarding certain perishables, a time that is peak ripeness for consumers should be a time when they eat the perishables. On the other hand, a time that is peak ripeness for producers should be after the perishables are shipped, sold and delivered to consumers. Also, although consumers may choose raw food or heating as ways of eating, there may be a case where producers and sellers cannot choose to heat or not depending on sales channels of the perishables. Hence, the condition receiving section 350 may receive information presenting types of users with designated information, and the proposed information specifying section 360 may specify proposed information according to the received types of users.

The perishables DB 370 manages information on perishables contained in the storage cabinet 100. The perishables DB 370 keeps information on perishables which are contained, and information such as determination methods for each information related to perishables according to types of perishables and causes of contamination.

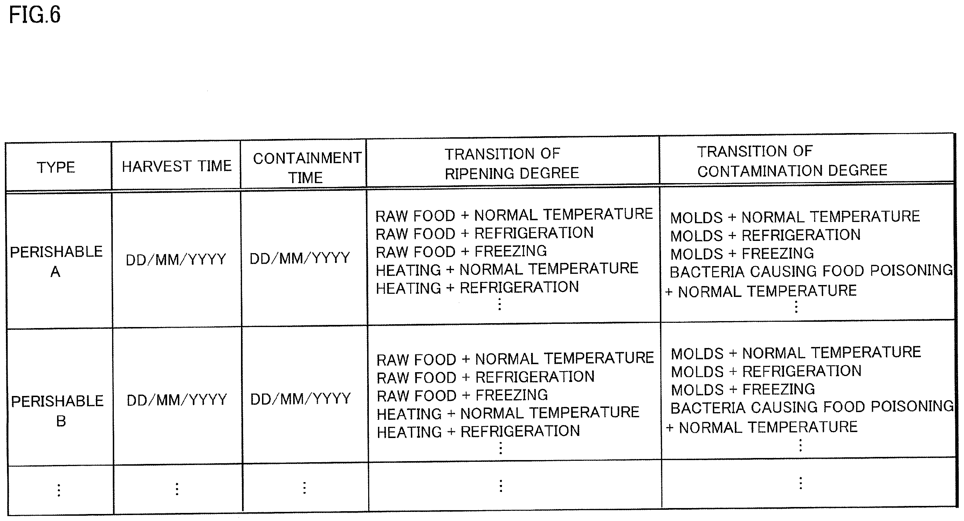

FIG. 6 is an example of a configuration of the perishables DB 370. Information on each item of types of perishables, harvest time, containment time, transition of ripening degree and transition of contamination degree is registered in the perishables DB 370 shown in the figure. As containment time, information on time when perishables were contained in the storage cabinet 100 is registered. As transition of ripening degree, information on transition of general ripening degree of subjected perishables is registered, for example, for each combination of ways of eating (raw food, heating and others) and preservation methods (normal temperature, refrigeration, freezing and others). As transition of contamination degree, information on transition of general contamination degree of subjected perishables is registered, for example, for each combination of causes of contamination (molds, bacteria causing food poisoning and others) and preservation methods (normal temperature, refrigeration, freezing and others). Determination by the determination section 340 is performed based on those information and information on current ripening degree and contamination degree specified by the ripening degree specifying section 320 and the contamination degree specifying section 330. It should be noted that as in the example above describing the proposed information specifying section 360, when different proposed information is specified according to types of users, information by types of users may be registered in the perishable DB 370 as information on transition of ripening degree and transition of contamination degree. In this case, the proposed information specifying section 360 specifies proposed information by using information on transition of ripening degree and transition of contamination degree corresponding to types of users received by condition receiving section 350.

Returning to FIG. 4, the determination result output section 380 outputs proposed information specified by the proposed information specifying section 360. Output proposed information is, for example, displayed by the display device 400 and presented to users. The determination result output section 380 is an example of an output section.

### <Inputs of designated conditions>

Inputs of designated conditions by users are done by the input device 500 shown in FIG. 1. For example, the perishable management device 300 displays a user interface (UI) screen for receiving inputs of designated conditions on the display device 400, and receives inputs of designated conditions made by users operating the input device 500 for the UI screen.

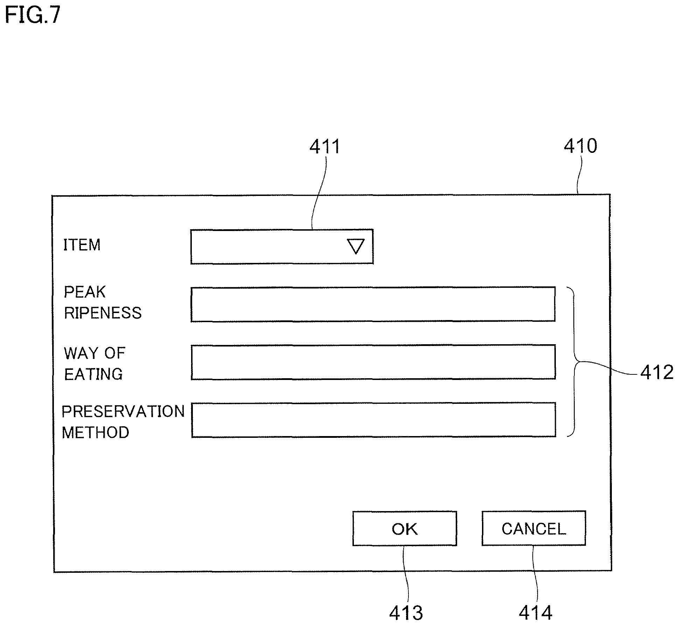

FIG. 7 is an example of a configuration of a UI screen for receiving inputs of designated conditions. A UI screen 410 shown in FIG. 7 displays a subject designation field 411, a condition input field 412 to input designated conditions, an OK button 413, and a cancel button 414. The subject designation field 411 is an object to designate items of perishables subjected to process. For example, a list of items of pre-registered perishables is displayed if users select the subject designation field 411 by a mouse pointer and touching. Users can designate perishables subjected to process by selecting desired items from the list of shown items. The list of items of perishables may be displayed as, for example, a pull-down menu. Items displayed on the list of items of perishables may be limited to perishables contained in the storage cabinet 100. In this case, if one type of perishables is contained in the storage cabinet 100, names of perishables contained in the storage cabinet 100 may be displayed as a text and others instead of the subject designation field 411. Items that can be input as designated conditions are displayed on the condition input field 412, and text boxes to input conditions are displayed for each item. Users select items that they want to designate as conditions and input the conditions into textboxes that correspond to items. The OK button 413 is a button object to instruct to confirm input designated conditions and send to the perishable management device 300. The cancel button 414 is a button object to instruct to move from the UI screen 410 without sending designated conditions to the perishable management device 300.

### <Outputs of proposed information>

When the perishables management device 300 receives designated conditions input from the UI screen 410 and others shown in FIG. 7 from the condition receiving section 350, the proposed information specifying section 360 specifies proposed information corresponding to designated perishables and designated conditions referring to the perishables DB 370. Then, the perishables management device 300 presents the proposed information to users by displaying a UI screen for presenting proposed information on the display device 400 by the determination result output section 380.

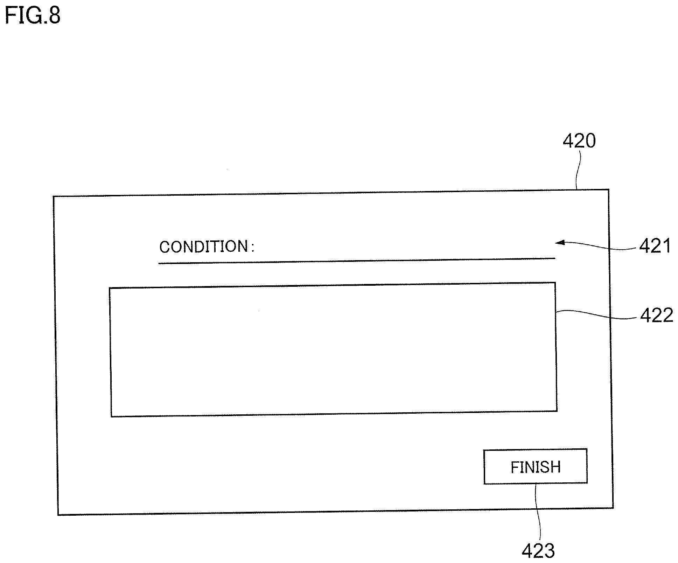

FIG. 8 is an example of a configuration of the UI screen for presenting proposed information. A UI screen 420 shown in FIG. 8 displays a condition display field 421, a proposed information display field 422, and a finish button 423. The condition display field 421 displays designated conditions utilized for the proposed information specifying section 360 of the perishables management device 300 to acquire proposed information. The proposed information display field 422 displays contents of proposed information specified by the proposed information specifying section 360. The finish button 423 is a button object to instruct to finish presenting proposed information and move from the UI screen 420.

While various embodiments have been described herein above, the technical scope of the present disclosure is not limited to the embodiments described above. For example, in the embodiments above, users input types of perishables subjected to process on the UI screen 410 when inputting designated conditions. On the other hand, types of perishables may be specified not by users' operations but by image recognition process. Also, specific examples of the storage cabinet 100 may be assumed to be buildings such as warehouses, houses and others as well as a refrigerator, and an aging cabinet used for food to ripen. Furthermore, the present disclosure includes many variations and alternations of configurations without departing from the scope of technical sprit in the present disclosure.

Here, the embodiments described above can be understood as below. The perishables management device 300 is a perishables management device 300 for information on perishables including: the ripening degree specifying section 320 configured to specify ripening degree of perishables; the contamination degree specifying section 330 configured to specify contamination degree of the perishables; and the determination result output section 380 configured to output information related to the perishables acquired by the ripening degree and the contamination degree of the perishables.

This perishables management device 300 can determine precisely states and handling of the perishables by using both ripening degree and contamination degree of perishables so that it is possible to detect matters that may be overlooked based only on either of them.

Here, the determination result output section 380 may be configured to output at least one of information related to peak ripeness of the perishables, information related to preservation of the perishables and information related to ways of eating the perishables.

This makes it possible to output information complementary related to "peak ripeness", "preservation methods" and "ways of eating" as information related to perishables.

Also, the determination result output section 380 may be configured to output information related to peak ripeness of the perishables, and/or information related to preservation of the perishables according to acquired information related to ways of eating the perishables.

This makes it possible to output information on "peak ripeness" and "preservation methods" complementary related to particular "ways of eating" regarding perishables.

Moreover, the determination result output section 380 may be configured to output information related to ways of eating the perishables and/or information related to preservation of the perishables according to acquired information related to time to eat the perishables.

This makes it possible to output information on "ways of eating" and "preservation methods" complementary related to particular "time to eat" regarding perishables.

Furthermore, the determination result output section 380 may be configured to output information related to peak ripeness of the perishables, and/or information related to ways of eating the perishables according to acquired information related to preservation methods of the perishables.

This makes it possible to output information on "peak ripeness" and "ways of eating" complementary related to particular "preservation methods" regarding perishables.

In addition, this perishable management device 300 may include: the condition receiving section 350 configured to acquire types of the perishables; wherein the determination result output section 380 is configured to output information related to the perishables according to types of the perishables acquired by the condition receiving section 350.

This makes it possible to determine precisely states and handling of the perishables based on ripening degree and contamination degree which are acquired according to types of perishables subjected to process, also for plural types of perishables.

Further, in this perishable management device 300, the ripening degree specifying section 320 may be configured to detect components related to ripening emitted by the perishables by the detection device 200 using biological components.

This makes it possible to detect components related to ripening emitted by the perishables with higher responsiveness compared to detections by electronic devices.

Also, in this perishable management device 300, the contamination degree specifying section 330 may be configured to detect components related to contamination emitted by the perishables by the detection device 200 using biological components.

This makes it possible to detect components related to contamination emitted by the perishables with higher responsiveness compared to detections by electronic devices.

Moreover, an information processing system of the present disclosure is an information processing system for perishables including: the ripening degree specifying section 320 configured to acquire ripening degree of perishables; the contamination degree specifying section 330 configured to acquire contamination degree of the perishables; and the determination result output section 380 configured to output proposed information on the perishables for users based on the ripening degree and the contamination degree of the perishables.

This information processing system can output more appropriately proposed information on the perishables for users by using both ripening degree and contamination degree of perishables so that it is possible to detect matters that may be overlooked based only on either of them.

Here, the determination result output section 380 may be configured to output the proposed information including different contents according to users.

This makes it possible to provide more appropriate information for each user even if a process is related to same perishables.

Furthermore, a program of the present disclosure is a program causing a computer to implement: a function of the ripening degree specifying section 320 configured to acquire ripening degree of the perishables; a function of the contamination degree specifying section 330 configured to acquire contamination degree of the perishables; and a function of the determination result output section 380 configured to output information related to the perishables acquired by the ripening degree and the contamination degree of the perishables.

A computer with this program installed can determine precisely states and handling of the perishables by using both ripening degree and contamination degree of perishables so that it is possible to detect matters that may be overlooked based only on either of them.

### Reference Signs List

- 10: Perishable management system
- 100: Storage cabinet
- 101: Door
- 102: Storage shelf
- 200: Detection device
- 300: Perishable management device
- 310: Data acquisition section
- 320: Ripening degree specifying section
- 330: Contamination degree specifying section
- 340: Determination section
- 350: Condition receiving section
- 360: Proposed information specifying section
- 370: Perishable database (DB)
- 380: Determination result output section
- 400: Display device
- 500: Input device

## Claims

1. An output device for information on perishables comprising:
a ripening degree specifying section configured to specify ripening degree of perishables;
a contamination degree specifying section configured to specify contamination degree of the perishables; and
an output section configured to output information related to the perishables acquired by the ripening degree and the contamination degree of the perishables.

2. The output device for information on perishables according to claim 1, wherein the output section is configured to output at least one of information related to peak ripeness of the perishables, information related to preservation of the perishables and information related to ways of eating the perishables.

3. The output device for information on perishables according to claim 2, wherein the output section is configured to output information related to peak ripeness of the perishables, and/or information related to preservation of the perishables according to acquired information related to ways of eating the perishables.

4. The output device for information on perishables according to claim 2, wherein the output section is configured to output information related to ways of eating the perishables, and/or information related to preservation of the perishables according to acquired information related to time to eat the perishables.

5. The output device for information on perishables according to claim 2, wherein the output section is configured to output information related to peak ripeness of the perishables, and/or information related to ways of eating the perishables according to acquired information related to preservation methods of the perishables.

6. The output device for information on perishables according to any one of claims 1 to 5 comprising:
an acquisition section configured to acquire types of the perishables; wherein
the output section is configured to output information related to the perishables according to types of the perishables acquired by the acquisition section.

7. The output device for information on perishables according to any one of claims 1 to 5, wherein the ripening degree specifying section is configured to detect components related to ripening emitted by the perishables by a sensor using biological components.

8. The output device for information on perishables according to any one of claims 1 to 5, wherein the contamination degree specifying section is configured to detect components related to contamination emitted by the perishables by a sensor using biological components.

9. An information processing system for perishables comprising:
a ripening degree acquisition section configured to acquire ripening degree of perishables;
a contamination degree acquisition section configured to acquire contamination degree of the perishables; and
an output section configured to output proposed information on the perishables for users based on the ripening degree and the contamination degree of the perishables.

10. The information processing system for perishables according to claim 9, wherein the output section is configured to output the proposed information including different contents according to the users.

11. A program comprising instructions which, when the program is executed by a computer, cause the computer to carry out;
a function configured to acquire ripening degree of perishables;
a function configured to acquire contamination degree of the perishables; and
a function configured to output information related to the perishables acquired by the ripening degree and the contamination degree of the perishables.
